# EUROPÄISCHE PATENTSCHRIFT

(11) **EP 2 277 038 B1**
(45) Veröffentlichungstag und Bekanntmachung des Hinweises auf die Patenterteilung: **11.09.2019**
(21) Anmeldenummer: 09745579.4
(22) Anmeldetag: 15.05.2009
(51) Int. Cl.: G01N 29/11, G01N 29/44

(54) **VERFAHREN ZUM IN-LINE MESSEN DES ERSTARRUNGS-, KONTRAKTIONS- SOWIE DES WANDABLÖSEVERHALTENS VON IN DER PRODUKTION IN FORMEN GEGOSSENER CONFECTIONARY-/SCHOKOLADENPRODUKTE UND VORRICHTUNG ZUM DURCHFÜHREN DIESES VERFAHRENS**
METHOD FOR THE IN-LINE MEASUREMENT OF THE SETTING, CONTRACTION AND WALL RELEASE BEHAVIOUR OF CONFECTIONERY/CHOCOLATE PRODUCTS WHICH HAVE BEEN POURED INTO MOULDS DURING PRODUCTION, AND APPARATUS FOR CARRYING OUT THIS METHOD
PROCÉDÉ DE MESURE EN LIGNE DU COMPORTEMENT DE SOLIDIFICATION, CONTRACTION ET DÉCOLLEMENT DES PAROIS DE PRODUITS DE CONFISERIE/CHOCOLATERIE COULÉS DANS DES MOULES LORS DE LEUR PRODUCTION ET DISPOSITIF POUR METTRE EN OEUVRE CE PROCÉDÉ

(30) Priorität: 16.05.2008 DE 102008024050
(43) Veröffentlichungstag der Anmeldung: 26.01.2011
(73) Patentinhaber: ETH Zurich, 8092 Zürich (CH)
(72) Erfinder: WINDHAB, Erich, J., CH-8261 Hemishofen (CH); MEHRLE, Yvonne, 66773 Schwalbach (DE); PFISTER, Bruno, CH-8194 Hüntwangen (CH)
(74) Vertreter: Beyer, Rudi
(86) Internationale Anmeldenummer: PCT/EP2009/003484
(87) Internationale Veröffentlichungsnummer: WO 2009/138246

(56) Entgegenhaltungen:
- EP-A- 0 577 511
- US-A1- 2003 051 535
- POVEY M J W: "ULTRASONICS OF FOOD" COMTEMPORARY PHYSICS, TAYLOR AND FRANCIS, GB, Bd. 39, Nr. 6, 1. November 1998 (1998-11-01), Seiten 467-478, XP009010723 ISSN: 0010-7514

## Beschreibung

Die Erfindung betrifft ein Verfahren zum in-line Messen des Verfestigungsverhaltens von in Formen gegossenen Confectionary-/Schokoladenprodukten.

Des Weiteren betrifft die Erfindung eine Vorrichtung zum Durchführen des Verfahrens.

Bei der Produktion stückiger Confectionary-/Schokoladenprodukte wird die noch flüssige Schokolade oder Füllmasse in Formen gefüllt und im weiteren Prozessverlauf einer Kühlungsbehandlung unterzogen. Die gefüllten Formen werden in einer Kühlkammer (batchweise) oder einem Kühltunnel (kontinuierlich) abgekühlt. Dabei erfolgt durch die einsetzende Verfestigungskristallisation der Fettkomponente, welche in der Regel die kontinuierliche zunächst fluide Suspensionsphase bildet, eine Volumenkontraktion, die in ihrer Ausprägung von Fettgehalt (i), Fettart/Fettmischung (ii) sowie Art und Grad einer eventuellen Vorkristallisation (iii) abhängt und das Produkt dadurch von der umgebenden Gießform ablösen lässt. Für eine fehlerfreie und homogen glänzende Schokoladenproduktoberfläche ist es wichtig, dass sich die Schokolade vor dem Austafeln gleichmäßig und gänzlich von der Formenwand ablöst, um keine Haftflecken/Kühlflecken oder in der Form wandanhaftend verbleibende Produktteile entstehen zu lassen.

Bisher wird der Zeitpunkt ab dem ohne Qualitätseinbußen ein Produkt ausgeformt werden kann, empirisch durch Probieren bestimmt. Dies führt zu langwieriger und Produktausschuss bedingende Einstellzeit des Kühlteils der Produktionsanlage. Änderungen von Produktrezeptur, Rohstoffen, Formengröße, Formenmaterial, Raum- /Umgebungstemperatur, Kühltemperatur, Kühlluftgeschwindigkeit und Verweilzeit in der Kühlstrecke sind Einflussgrößen, welche die Kinetik der Abkühlungs-, Erstarrungs- und Wandablösevorgänge maßgeblich beeinflussen.

Literatur- und Patentrecherchen haben ergeben, dass bislang keine erfindungsgemäß realisierte in-line Messvorrichtung zur Ermittlung des Erstarrungs-, Kontraktions- und Formenwandablöseverhaltens von Schokoladen oder schokoladenähnlichen Produkten beschrieben ist. In der Schokoladenindustrie werden z. T. optische Messgeräte eingesetzt, die nach erfolgter Ausformung Produkte aussortieren, welche den gewünschten Oberflächenqualitätskriterien nicht entsprechen. Aufgrund der Anzahl aussortierter Teile pro Zeiteinheit wird beispielsweise der Durchsatz über die Geschwindigkeit des Formentransportbandes verändert.

In der Spritzgusstechnik wird an ein oder zwei Orten in der Gießform mittels Druckmessung festgestellt, wann sich das Bauteil an dieser Stelle von der Wand gelöst hat. Bei diesem Verfahren wird nur der Zeitpunkt des Ablösens an einer (bzw. zwei) Stelle(n) bestimmt. Des Weiteren besteht die Oberfläche des Drucksensors aus einem anderen Material, als die Gießform selbst, was bewirkt, dass der Ablöseprozess von der Druckaufnehmeroberfläche nicht repräsentativ für das Ablöseverhalten vom Formenwandmaterial ist. Für Confectionary Produkte ist es ferner unerwünscht, wenn anstelle einer einheitlich glänzenden Produktoberfläche, Abdrücke von Sensoreinbauten sichtbar würden.

Ultraschall wird als Basis für Messtechniken zur Ermittlung von Materialstrukturen in verschiedenen Industriebereichen verwendet. Beispielsweise wird das Ultraschallreflexionsverhalten an Materialien unterschiedlicher Dichte bzw. unterschiedlicher Schallgeschwindigkeiten genutzt, um Materialfehler u. a. in metallischen Werkstoffen zu detektieren /1-3/. In der Bauindustrie kommt Ultraschallmesstechnik zum Einsatz, um Betonwände auf Lunker (eingeschlossene Lufthohlräume) zu untersuchen. In der Medizintechnik werden Gewebestrukturen unterschiedlicher Dichte aus diagnostischen Gründen visualisiert /4/. Im Falle bewegter Medien/Objekte können Ultraschall-Doppler Verfahren Geschwindigkeitsfelder bzw. Volumenströme detektieren lassen /5/. Die Kopplung von Ultraschall-Doppler und Druckmesstechnik ermöglicht in-line Messungen komplexer rheologischer Eigenschaften /6,7/.

Aus der FR 2 656 425 A1 ist ein Verfahren zum zerstörungsfreien Prüfen von Betonelementen vorbekannt.

"R. Saggin, et. Al. "Measurement of solid fat content by ultrasonic reflectance in model systems and chocolate", Food Research International 35 (2002) S. 999-1005" betrifft die Kontrolle von Inhaltsstoffen bei der Herstellung von Schokoladenprodukten durch Messung des Fettgehalts (solid fat content - FSC). Durch die Messung des FSC-Gehalts lässt sich das Ausformverhalten von Confectionary/Schokoladeprodukten nicht bestimmen.

Die US 2006/0123914 A1 betrifft die Messung des Aushärteverhaltens von Kunststoffen. Auf die Besonderheiten des Ausformverhaltens von Confectionary/Schokoladeprodukten nimmt diese Literaturstelle nicht Bezug.

Die EP 0 577 511 A beschreibt ein Verfahren zum in-line-Messen des Erstarrungsverhaltens von Lebensmitteln, die sich in einem Behälter befinden, unter Verwendung von Ultraschallsensoren (Sender und Empfänger), wobei kein direkter Sensorkontakt zum Lebensmittel besteht und aus dem gesamten und von dem Empfänger empfangenen Schwingungssignal die Amplitudendämpfung ermittelt wird und damit der Zeitpunkt der Erstarrung des Lebensmittels bestimmt wird. Auch die Anwendung einer Kalibrierung wird erwähnt.

Die US 2003/051535 A1 betrifft ein Verfahren zum Messen der physikalischen Eigenschaften von fluiden Massen mittels Ultraschall, unter anderem von Nahrungsmitteln. Die von einem Sender durch das fluide Material gesandten Wellen werden von einem Empfänger aufgefangen und sollen Aufschluss über die charakteristischen Eigenschaften geben. Genannt werden unter anderem Sodium Chloride, Glycerol, Tomatenketchup und Überzüge von Confectionaryprodukten wie Kakaobutter sowie Fette. Diese Druckschrift bezieht sich nicht auf die Bestimmung des Ausformzeitpunktes, sondern aus dem gesamten und von dem Empfänger empfangenen Schwingungssignal werden die Schallgeschwindigkeit und der Reflexionskoeffizient ermittelt, um daraus physikalische Eigenschaften des Produktes zu bestimmen.

Povey M.J.W. "Ultrasonics of Food" Contemporary Physics, Tayler and Francis, GB, Bd. 39, Nr. 6, 1. November 1998, Seiten 467-478, ISSN: 0010-7514 offenbart ein Verfahren zum Messen des Erstarrungsverhaltens von Schokolade, die sich in einem Behälter befindet, unter Verwendung von Ultraschallsensoren (Sender und Empfänger), wobei kein direkter Sensorkontakt zum Lebensmittel besteht. Aus dem gesamten und von dem Empfänger empfangenen Schwingungssignal wird die Schallgeschwindigkeit ermittelt, um den Erstarrungszeitpunkt zu bestimmen. Die Druckschrift bezieht sich aber nicht auf die Bestimmung des Ausformzeitpunktes.

### Aufgabe

Der Erfindung liegt die Aufgabe zugrunde, ein Verfahren zum in-line Messen des Erstarrungs-, Kontraktions- sowie des Wandablöseverhaltens von in Formen gegossener Confectionary-/Schokoladeprodukte zu schaffen, um den Ausformzeitpunkt von in Gießformen gegossener, erstarrender Produkte aus diesen Stoffen optimal bestimmen zu können.

Des weiteren liegt der Erfindung die Aufgabe zugrunde, zur Durchführung des Verfahrens eine vorteilhafte Vorrichtung vorzuschlagen.

### Lösung der Aufgabe betreffend das Verfahren

Diese Aufgabe wird durch die in **Patentanspruch 1** wiedergegebenen Merkmale gelöst.

### Einige Vorteile

Mit der erfindungsgemäßen, innovativen in-line Messvorrichtung ist es nun erstmals möglich, den Verlauf von Abkühlung (i), Erstarrung (ii) und Produktablösung von der Formenwand (iii) exakt und in-line zu ermitteln. Dies schafft erstmals die Möglichkeit eine optimierte Auslegung des Kühlprozesses unter Berücksichtigung aller vorab genannten Einflussparameter zielgerichtet vorzunehmen. Optimierungskriterien sind: (i) die exakte Festlegung des Ausformzeitpunktes aus der Gießform, (ii) dessen weitest gehende Verkürzung unter (iii) Gewährleistung einer optimalen, optisch homogenen, glänzenden Produktoberfläche vorzunehmen. Gegenüber dem bisherigen Stand der Technik, welcher eine in-line Detektion des Verfestigungs- und Formenwand-Ablöseverhaltens bislang nicht erlaubt, bedeuten erfindungsgemäße Vorrichtung und Verfahren (a) eine Reduktion von Fehlproduktionen, (b) verbesserte Produktqualitätskonstanz und (c) verlängerte Lebensdauer der Gießformen, was eine deutliche Kostenreduktion und ein erweitertes Marktpotential realisieren lässt.

Beim Abkühlen von in Formen gegossenen Confectionary/Schokoladeprodukten kann deren Verfestigungsvorgang sowie deren auf Grund produkttypischer Volumenkontraktion erfolgendes Ablösen von der Gießformenwand, bislang nicht während des Abkühlvorganges in-line ermittelt werden. Die Konsequenz für Hersteller solcher Produkte sind entweder zu lange Verweilzeiten im Kühl-/ Erstarrungsprozess mit resultierend nur teilgenutzter Produktionskapazität (i) oder zu kurze Verweilzeiten mit in der Folge resultierendem Teilanhaften vom Produkt an der Formenwand und somit schlechtem Auslösen desselben aus der Gießform im Verbund mit Produktschädigung (Oberflächenfehler) und Formenverschmutzung (ii). Bei der Erfindung werden Erstarrungs- und Wandablösevorgänge während des Kühl-/Erstarrungsschrittes in der Produktion gegossener Confectionary/Schokoladenprodukte in-line gemessen, um eindeutige Kriterien für die optimierte Einstellung von Kühl-/ Erstarrungsprozessparametern wie z. B. Kühltemperatur, Kühlluftgeschwindigkeit, Verweilzeit in der Kühlstrecke (z. B. bestimmt über Bandgeschwindigkeit) zu liefern. Derartige Informationen werden entweder telemetrisch an einen Steuer-/Regelrechner oder externen Computer übertragen oder nach Durchlauf einer mit entsprechender Messtechnik und Elektronik bestückten Gießform via Computer ausgelesen. Auf der Basis der erfindungsgemäßen in-line Messtechnik lässt sich die Produktqualität optimieren.

Somit ermöglicht die erfindungsgemäße Vorrichtung die nicht invasive Messung der Verfestigungseigenschaften und des Ablösezeitpunkts von in Formen gegossenen Massen, welche in einem geeigneten Prozess, z. B. einem Kühlprozess der Verfestigung/Erstarrung unterzogen werden. Aus dem charakteristischen Messkurvenverlauf werden Grad der Verfestigung und der Ablösung der erstarrten Masse von der Formenwand genau bestimmbar.

Das erfindungsgemäße Verfahren nutzt den Einbau der erfindungsgemäßen Messvorrichtung in eine Gießform, verfolgt das Verfestigungs-/Erstarrungsverhalten der in die Form gegossenen Masse in-line während des Kühlprozesses und übermittelt die entsprechenden Daten an einen Prozessrechner. Damit wird der optimale Zeitpunkt für die Ausformung exakt ermittelt und minimale Prozesszeit sowie optimale Produktqualität über eine mit dem Prozessrechner gekoppelte Regelung, welche die Stellgrößen: Kühltemperatur, Kühlluftgeschwindigkeit und Bandgeschwindigkeit berücksichtigt, erzielt.

### Weitere erfinderische Ausgestaltungen

Weitere erfinderische Ausgestaltungen sind in den **Patentansprüchen 2** bis **4** beschrieben.

### Lösung der Aufgabe hinsichtlich die Vorrichtung

Diese Aufgabe wird durch die Merkmale des **Patentanspruches 5** gelöst.

### Weitere erfinderische Ausgestaltungen

Weitere erfinderische Ausgestaltungen zur Vorrichtung sind in den **Patentansprüchen 6** und **7** beschrieben.

In der Zeichnung ist die Erfindung beispielsweise dargestellt. Es zeigen:
- Fig. 1: eine Formenwand von der Unterseite her gesehen;
- Fig. 2: ein Amplituden-Zeitdiagramm mit Ultraschall-Sendesignal und Ultraschall-Empfängersignal;
- Fig. 3: ein Amplituden-Zeitdiagramm für Milchschokolade
- Fig. 4: ein Amplituden-Zeitdiagramm mit dem Temperaturverlauf in zu verfestigender Schokolade im Vergleich zum Amplitudenverlauf als Funktion der Zeit, und
- Fig. 5: ein Amplituden-Zeitdiagramm mit verschiedenen Kühltemperaturen bei Milchschokolade.

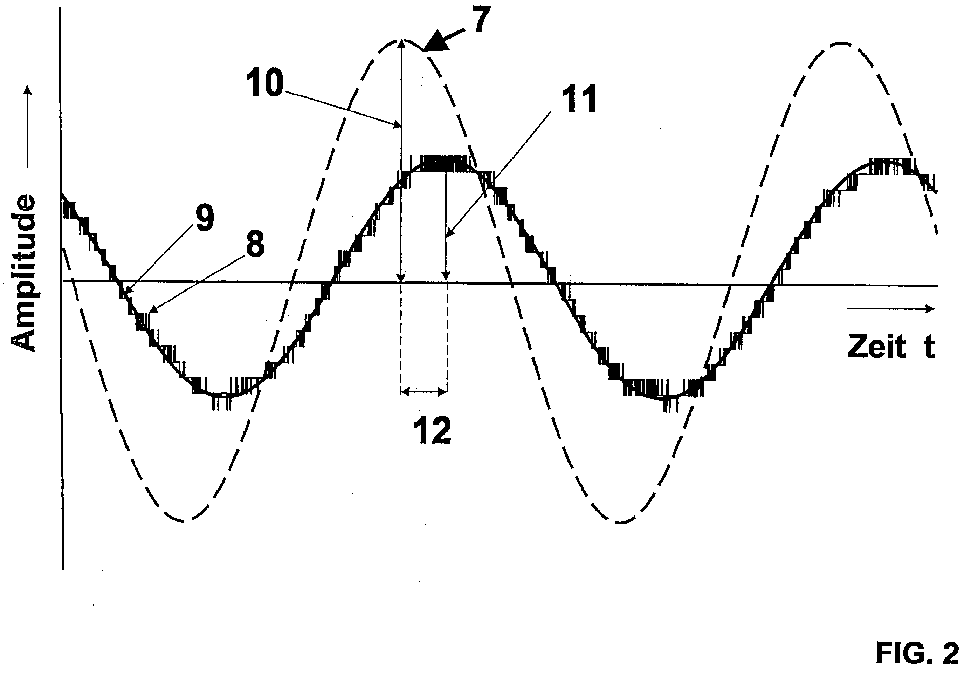

- Fig. 2: ein Amplituden-Zeitdiagramm mit Ultraschall-Sendesignal und Ultraschall-Empfängersignal;

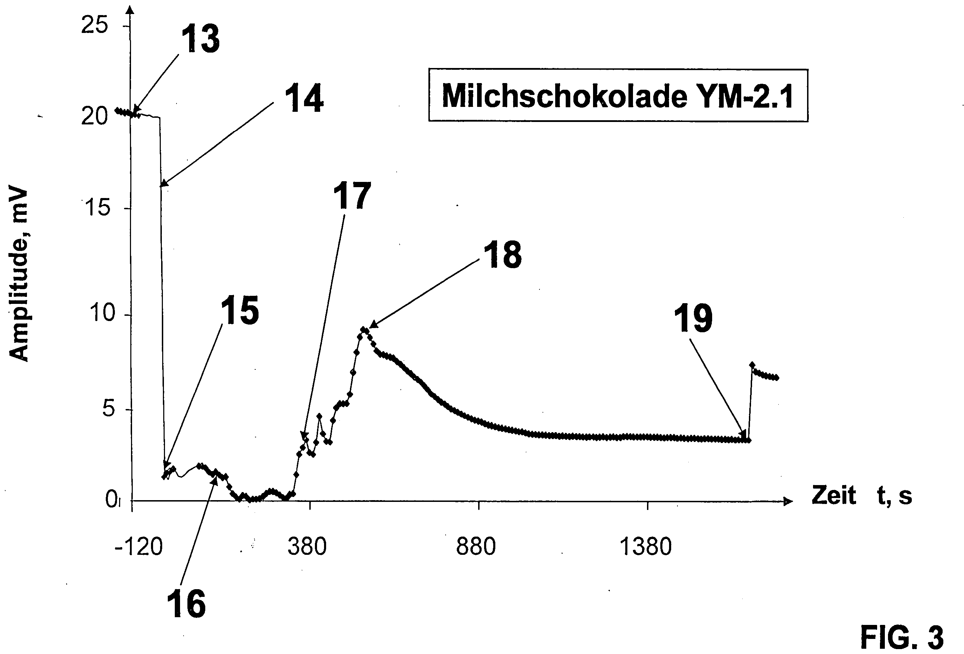

- Fig. 3: ein Amplituden-Zeitdiagramm für Milchschokolade;

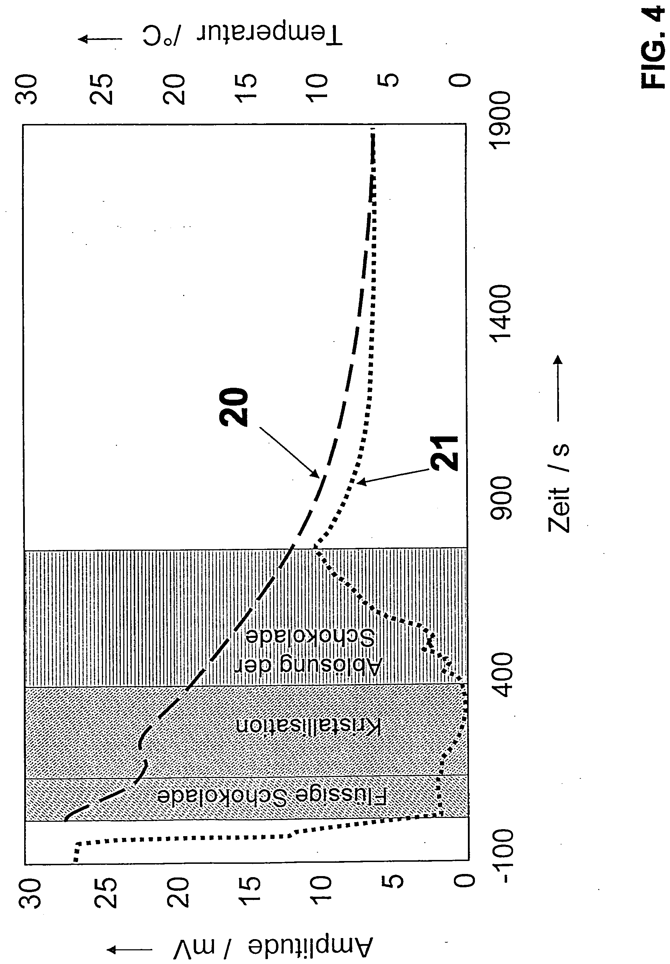

- Fig. 4: ein Amplituden-Zeitdiagramm mit dem Temperaturverlauf in zu verfestigender Schokolade im Vergleich zum Amplitudenverlauf als Funktion der Zeit, und

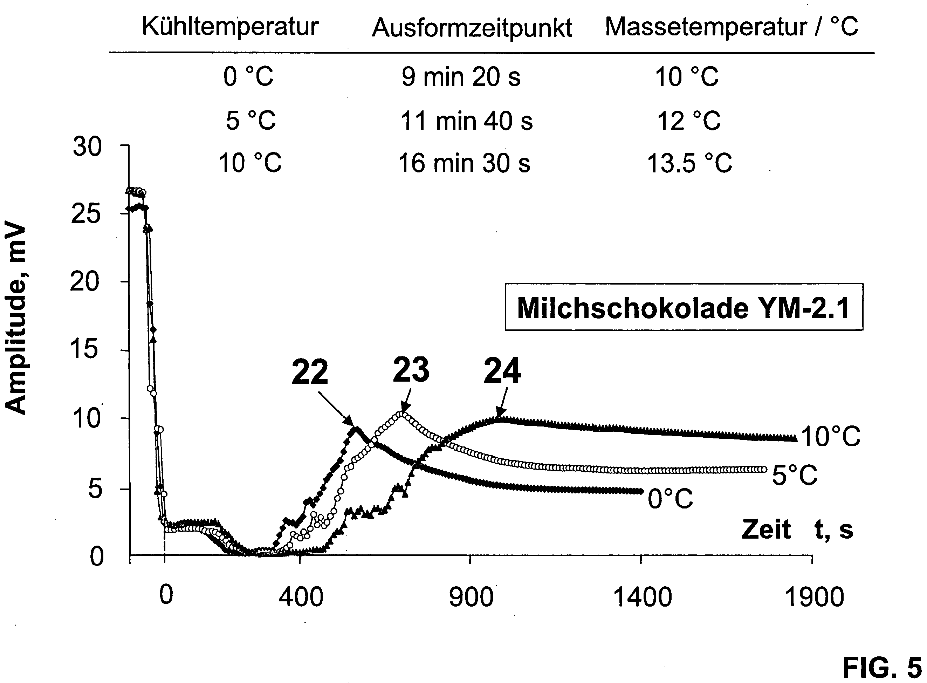

- Fig. 5: ein Amplituden-Zeitdiagramm mit verschiedenen Kühltemperaturen bei Milchschokolade.

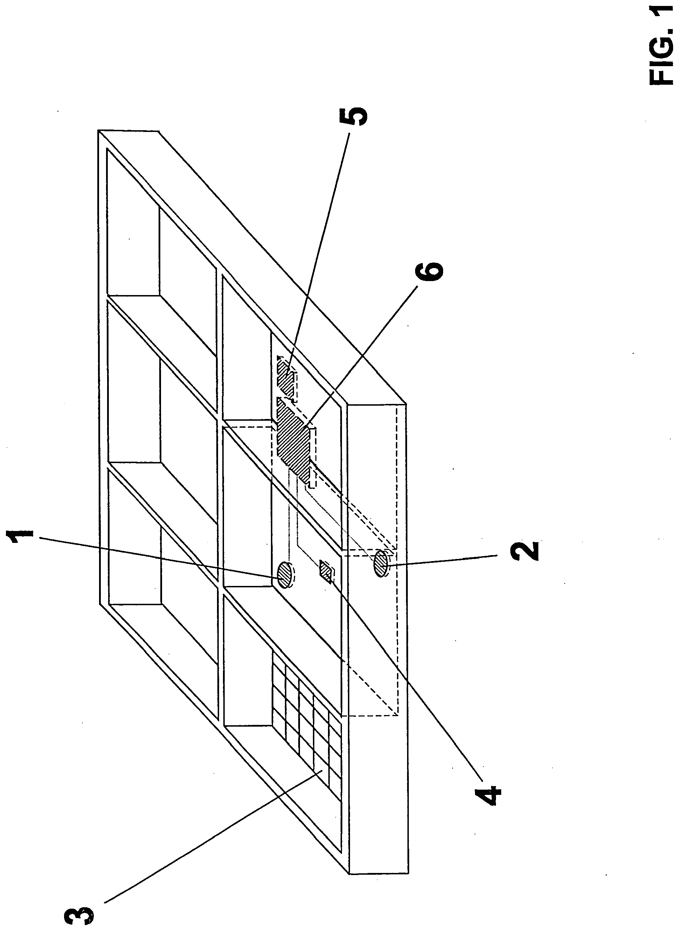

### a) Geometrie / Anordnung (Fig. 1)

Die Messvorrichtung besteht aus zwei Ultraschallsensoren (Sender 1 und Empfänger 2), welche mit der Wand der zu befüllenden Gießform 3 derart verbunden werden, dass kein direkter Sensorkontakt zum Gießmaterial entstehen kann. In der Regel bedeutet dies, dass eine wandrückseitige Installation erfolgt. Der Abstand der beiden Sender und Empfänger (Sensoren) wird bevorzugt so gewählt, dass eine möglichst lange von Gießmaterial überdeckte Strecke in einer Gießform dazwischen liegt.

Auf der Rückseite der "Mess-Gießform" sind des weiteren ein Mikrocomputer/Mikroprozessor 6 in wasserfestem Gehäuse sowie eine diesen mit Spannung versorgende Batterie-Spannungsquelle 5 mit der Form fest verbunden, angeordnet. Zur Messung der Massetemperatur an der Formenwand ist zusätzlich die Integration von Thermoelementen 4, montiert von der Formenrückseite, gegebenenfalls mit Gießmassekontakt, vorgesehen.

### b) Funktionsweise (Fig. 2, 3)

Mittels Signalgenerator wird der Ultraschallsender 1 angeregt und aus gesandtem und vom Empfänger 2 empfangenem Schwingungssignal die Amplitudendämpfung sowie die Phasenverschiebung der leeren Form bei Raumtemperatur ermittelt. Somit ist ein formenspezifisches Kalibriersignal gespeichert. Danach kann die Form im Gießprozess eingesetzt werden.

Die gesendeten Ultraschallwellen 7 breiten sich im Gießformenmaterial und der gegebenenfalls eingegossenen Masse (z. B. Schokolade) aus und werden vom Empfänger 2 detektiert. Dabei kommt es zu Energieverlusten in Folge Energieabsorption, d. h. dissipativen Umwandlung von Schwingungsenergie in molekulare Reibung im zur Schwingung mit angeregten Material, was zur Abschwächung des Empfänger-Amplitudensignals 8, 9 (Amplitudendämpfung 10, 11) und dessen Phasenverschiebung 12 führt. Je viskoser das in Schwingung versetzte Material ist, umso stärker erfolgt die dissipative Amplitudendämpfung und Phasenverschiebung. Je elastischer dasselbe ist, umso entsprechend weniger sind Amplitudendämpfung und Phasenverschiebung ausgeprägt. Neben der Materialart der Gießform nimmt das mit in Schwingung versetzte, an der Formenwand anhaftende Material des in die Form eingegossenen Produktes Einfluss auf die dämpfungsbedingte Veränderung der Schwingung. Somit wird letztere auch von der Art des eingegossenen Materials, seiner Viskosität sowie der Temperatur und der Festigkeit des Haftverbundes zwischen Formen- und Gießmaterial abhängig. Liegt ein fester Haftverbund zwischen Formenwand- und Gießmaterial vor, und besitzen diese beiden Materialien eine vergleichbare Schallgeschwindigkeit so kann die Ultraschallwelle verlustarm von Formenwandmaterial ins Gießmaterial übertreten. Herrschen allerdings große Unterschiede in der Schallgeschwindigkeit zwischen benachbarten Materialphasen, wie dies beispielsweise bei Ausbildungen eines, wenngleich sehr engen Luftspaltes zwischen der Gießformenwand und dem Gießmaterial nach dessen Wandablösung der Fall ist, so werden die Ultraschallwellen an der Phasengrenzfläche verstärkt reflektiert. Damit wird das Gießmaterial nur noch reduziert in die Schwingung mit einbezogen.

Beim Abkühlen von Schokolade bzw. schokoladenartigen Massen als Gießmaterial und typischen Kunststoffformen (Macrolon) als Wandmaterial erfolgt bei einsetzender Verfestigungskristallisation der Schokolade deren bis ca. 1-3%-ige Volumenkontraktion. Diese führt schließlich zur Ablösung des Gießmaterials von der Formenwand. Dabei entsteht zwischen Formwand und Schokolade ein dünner Luftspalt, an dem die Ultraschall-Welle teilreflektiert wird, was eine Reduktion der Amplitudendämpfung nach sich zieht, d. h. für das empfangene Signal steigt die Amplitude in Folge reduzierter Dämpfung. Da die Ablösung von der Formenwand lokal beginnt und voranschreitet bis die vollständige Ablösung erfolgt, ist von einer mehr oder weniger stetigen Zunahme des empfangenen Amplitudensignals über diesen Zeitraum auszugehen.

Die Schokolade durchläuft während des Abkühlprozesses verschiedene "Konsistenz-Stadien", von flüssig über pastös bis fest. Da - wie vorbeschrieben - die Dämpfung des Ultraschallsignals von Viskosität, Temperatur und Haftverbund Gießmaterial/Formmaterial abhängt, repräsentiert das empfangene Schallsignal eine Überlagerung aller dieser Abhängigkeiten. Wie jedoch überraschenderweise gefunden werden konnte, zeigt sich sobald die Schokolade von der Wand löst, dass der damit verbundene Reduktionseffekt der Amplitudendämpfung die anderen Einflüsse eindeutig dominiert. Damit kann aus dem Maximum des empfangenen Amplitudensignals (Fig. 3, 18) eindeutig der relevante Zeitpunkt der vollständigen Ablösung der Gießmasse von der Formenwand eingegrenzt werden. Dieser Zeitpunkt markiert die benötigte Kühldauer, um ein optimal glänzendes Oberflächenergebnis zu erzielen. Dies wurde mittels Austafeltests bestätigt, die zu verschiedenen Zeitpunkten durchgeführt wurden.

Nach Überschreiten des derart ermittelten Amplitudenmaximums (= Dämpfungsminimums) bewirkt der weitergehende Abkühlvorgang des Formmaterials eine stetige Amplitudenreduktion (Fig. 3, 19). Im Falle Auftreten von "Zwischenmaxima" (Fig. 3, 17) während des fortschreitenden Ablösevorganges der Gießmasse von der Formenwand, ist die Stetigkeit des einem solchen Zwischenmaximum nachfolgenden Signals in aller Regel nicht gegeben, d. h. es folgt ein unstetiger Kurvenverlauf der Amplitudenfunktion über der Zeit in Folge weiterschreitender Teilablösung von der Formenwand. Zeigt das Folgesignal nach Auftreten eines Maximums jedoch stetigen Verlaufscharakter, z. B. eine weiterschreitende stetige Abnahme in Folge weitergehender Abkühlung, dann ist dies - wie gezeigt werden konnte - ein hinreichendes Maß dafür, dass das betreffende Amplitudenmaximum dem Punkt der vollständigen Ablösung entspricht.

### c) Messergebnisse (Fig.3, 4, 5)

Die Fig. 3 bis 5 zeigen zeitliche Amplitudenverläufe (Empfänger) als Funktion der Prozesszeit für in Macrolon Tafelformen nach Vorkristallisation (Temperierung) ausgegossene Milchschokolade (YM-2.1). In Fig. 4 ist der Temperaturverlauf in der Gießmasse zusätzlich integriert. Fig. 5 demonstriert den Einfluss verschiedener Kühltemperaturen (Kühlluftstrom).

Für die leere Gießform unter Raumtemperaturbedingungen zeigt sich eine Empfänger-Amplitude von ca. 20 mV (Fig. 3, 13), welche während des Einfüllvorganges der Milchschokoladenmasse bei ca. 28°C bis zur vollständigen Füllung der Form auf ca. 1.7 mV abnimmt (Fig. 3, 15). Die danach einsetzende Kühlung (in Fig. 3 mit 10°C Kühltemperatur) bewirkt ein Voranschreiten der Verfestigungskristallisation und damit eine Erhöhung der Masseviskosität mit in der Folge weitergehend verstärkter Amplitudendämpfung (bis ca. 0.023 mV Amplitudensignal). Nach ca. 380 Sekunden ab Kühlbeginn ist die Masse in der Gießform weitgehend erstarrt, und in Folge der damit verbundenen Massekontraktion beginnt die Ablösung von der Formenwand. Dies bewirkt die fortschreitende in der Regel nicht gleichmäßig voranschreitende Luftspaltausbildung zwischen Formwand und Masse und somit nicht eine stetig abnehmende Amplitudendämpfung (Fig. 3, 17). Ein Maximum der Amplitude bei ca. 550 s mit nachfolgend deutlich verbessert stetig fortschreitende Abnahme entspricht der vollständigen Masseablösung von der Formenwand (Fig. 3, 18) und damit den idealen Ausformungszeitpunkt. Die Amplitudenabnahme nach Durchschreiten dieses Maximums im Amplitudenverlauf kann der weiteren Abkühlung des Systems zugeschrieben werden. Dies wird in Fig. 4 durch den zusätzlich synchron zum Amplitudenverlauf dargestellten zeitlichen Temperaturverlauf in der abkühlenden Masse (Fig. 4, 20) demonstriert. Die entsprechende Temperaturmessung erfolgte in der Masse mittels integriertem Thermoelement (vgl. Fig. 1, 4).

Fig. 5 zeigt eindrücklich auf, dass eine auf breiter Skala variierte Kühltemperatur (hier zwischen 0 und 10°C) einen in der charakteristischen Form gleichartigen zeitlichen Amplitudenverlauf resultieren lässt, aus dessen jeweiligen Maximum nach Kühlbeginn die optimalen Ausform-/Austafelungszeiten bei entsprechender Kühltemperatur eindeutig zu entnehmen sind (Fig. 5, 22, 23, 24, siehe auch Tabelle in Fig. 5). Die zeitlichen Verläufe der Amplituden- und Temperaturfunktionen können mittels drahtloser Verbindung (wireless) aus dem Prozess praktisch verzögerungsfrei direkt an einen Prozessrechner oder externen Computer übertragen werden. Alternativ kann eine Zwischenspeicherung auf einem elektronischen Baustein erfolgen und nach Entnahme der Mess-Gießform aus dem Prozess (nach Ausformung) via Kabelverbindung ein Auslesen der Daten mittels Computer erfolgen.

### Bezugszeichen

- 1: Ultraschall-Sender
- 2: Ultraschall-Empfänger
- 3: Einzel-Giessform
- 4: Thermoelement (Temperaturmessung)
- 5: Energiequelle (Batterie oder Akku)
- 6: Mikroprozessor, Signalverarbeitung, Speicher, (Funk-) Wireless/Telemetrie Verbindung
- 7: Ultraschall Sendesignal
- 8: Ultraschall Empfängersignal
- 9: Sinuskurven-Fit für Empfängersignal
- 10: Ultraschall Sendesignal - Amplitude
- 11: Ultraschall Empfängersignal - Amplitude
- 12: Phasenverschiebung zwischen Sender- und Empfängersignal
- 13: Ultraschall-Empfängersignal-Amplitude (leere Form, Raumtemperatur)
- 14: Ultraschall-Empfängersignal-Amplitude (bei Formfüllen)
- 15: Ultraschall-Empfängersignal-Amplitude (gefüllte Form, Kühlbeginn)
- 16: Ultraschall-Empfängersignal-Amplitude (Viskositätserhöhung durch Kristallisation)
- 17: Ultraschall-Empfängersignal-Amplitude (Teilablösung von Formenwand)
- 18: Ultraschall-Empfängersignal-Amplitude (vollständige Ablösung von Formenwand)
- 19: Ultraschall-Empfängersignal-Amplitude (Leeren der Form, Austafeln)
- 20: Temperaturverlauf in der verfestigenden Masse (hier Schokolade) als Funktion der Zeit
- 21: Amplitudenverlauf als Funktion der Zeit
- 22: Amplitudenverlauf als Funktion der Zeit bei 0°C Kühltemperatur
- 23: Amplitudenverlauf als Funktion der Zeit bei 5°C Kühltemperatur
- 24: Amplitudenverlauf als Funktion der Zeit bei 10°C Kühltemperatur

### Literaturverzeichnis

**/1/** Rehm, G., Waubke, N. V., Neisecke, J.: Ultraschall-Untersuchungsmethoden in der Baupraxis - Literatursichtung. Berichte aus der Baupraxis 84 (1973), S. 3-23.
**/2/** Teodoru, G.: Zerstörungsfreie Betonprüfung: insbesondere Anwendung von Ultraschall; kritische Betrachtungen. Düsseldorf: Beton-Verl., 1989.
**/3/** Popovics, J. S., Rose, J. L.: A survey of developments in ultrasonic NDE of concrete. IEEE Trans. on Ultras., Ferroelectr. and Freq. Contr. 41 (1994), Nr. 1, S. 140-143.
**/4/** Berger, R. (2000). Moderne bildgebende Verfahren der medizinischen Diagnostik - ein Weg zu interessanterem Physikunterricht. Diss. Universität München. Berlin: Logos Verlag
**/5/** Baker D. W. et al. (1978)Doppler principles and techniques; Ed. Fry, F.T., Amsterdam, 219-254.
**/6/** Ouriev, B., and Windhab, E. (2003). "Transient Flow of Highly Concentrated Suspensions Investigated Using the Ultrasound Velocity Profiler-Pressure Difference Method." Measurement Science & Technology, 14(11), 1963-1972.
**/7/** Birkhofer, B., Jeelani, S. A. K., Ouriev, B., and Windhab, E. J. (2006). "In-line characterization and rheometry of concentrated suspensions using ultrasound." Ultrasound 06, Leeds, UK, February 8-10.
**/8/** FR 2 656 425 A1
**/9/** R. Saggin, et. al., "Measurement of solid fat content by ultrasonic reflectance in model systems and chocolate", Food Research International 35 (2002), S.999-1005
**/10/** US 2006/0123914 A1

## Patentansprüche

1. Verfahren zum in-line-Messen des Verfestigungsverhaltens von in Formen gegossenen Confectionary-/Schokoladenprodukten während des Kühlprozesses zur Ermittlung des optimalen Zeitpunkts für die Ausformung des Fertigproduktes unter Verwendung mindestens eines Ultraschallsenders (1) und wenigstens eines Ultraschallempfängers (2) als Ultraschallsensor, wobei kein direkter Kontakt zum Confectionary-/Schokoladenprodukt besteht, und aus ausgesandtem und von dem Ultraschallempfänger (2) empfangenen Schwingungssignal (7) die Amplitudendämpfung (11, 14) der leeren Gießform (3) ermittelt wird, wobei ein gießformspezifisches Kalibriersignal in einem Prozessrechner oder Computer gespeichert wird, woraufhin die Gießform (3) für den Gießprozess des Confectionary-/Schokoladenprodukts eingesetzt wird und der vom Ultraschallsender (1) über das Gießformmaterial und das als Confectionary-/Schokoladenprodukt ausgebildete Gießmaterial sich ausbreitende Ultraschall (14) von dem Ultraschallempfänger (2) detektiert wird und aus der dissipativen Amplitudendämpfung und der Phasenverschiebung (12) des Ultraschalls aus dem Maximum (7) der relevante Zeitpunkt der vollständigen Ablösung des Confectionary-/Schokoladenprodukts von der Gießformwand eingegrenzt wird mit nachfolgender deutlicher, stetig fortschreitender Abnahme des Amplitudensignals und damit der Zeitpunkt der Ausformung des Confectionary-/Schokoladenprodukts bestimmt wird.

2. Verfahren nach Anspruch 1, **dadurch gekennzeichnet, dass** die zeitlichen Verläufe der Amplituden- und Temperaturfunktionen mittels drahtloser Verbindung aus dem Gießprozess praktisch verzögerungsfrei direkt an einen Prozessrechner oder externen Computer übertragen werden.

3. Verfahren nach Anspruch 1, **dadurch gekennzeichnet, dass** eine Zwischenspeicherung der zeitlichen Verläufe der Amplituden- und Temperaturfunktionen aus dem Gießprozess auf einen elektronischen Baustein erfolgen und nach Entnahme der Mess-Gießform aus dem Gießprozess - nach Ausformung - über eine Kabelverbindung ein Auslesen der Daten mittels Computer erfolgt.

4. Verfahren nach Anspruch 1 oder einem der darauf folgenden Ansprüche, **dadurch gekennzeichnet, dass** die in-line ermittelten Daten zum Erstarrungs- /Verfestigungsverhalten der Confectionary-/Schokoladenmassen, das Wandablöseverhalten und der Zeitpunkt der vollständigen Wandablösung zur Steuerung oder Regelung der den Verfestigungsprozess beeinflussenden Prozessparameter wie Kühltemperatur, Kühlluftgeschwindigkeit und Bandgeschwindigkeit zur Erzielung minimaler Verweilzeit in dem Prozess und/oder zur Qualitätsoptimierung der Produkteigenschaften genutzt werden.

5. Vorrichtung zum Durchführen des Verfahrens nach einem der Ansprüche 1 bis 4, mit einer Gießform (3) für Confectionary-/Schokoladenprodukte als Gießmaterial, mindestens einem Ultraschallsender (1) und wenigstens einem Ultraschallempfänger (2) zum Empfang der durch das Gießformmaterial und berührende Confectionary-/Schokoladenmasse teilweise gedämpften Schwingung, wobei der Ultraschallsender und der Ultraschallempfänger an der das erstarrende Gießmaterial begrenzenden Gießformwand angeordnet sind, und einem Prozessrechner oder externen Computer, eingerichtet zum Durchführen des genannten Verfahrens.

6. Vorrichtung nach Anspruch 5, **dadurch gekennzeichnet, dass** die anregende Schwingungsfrequenz des betreffenden Ultraschallsenders auf die Resonanzfrequenz der die erstarrende Confectionary-/Schokoladenmasse begrenzenden Wand abgestimmt ist.

7. Vorrichtung nach Anspruch 5 oder 6, **dadurch gekennzeichnet, dass** der Abstand zwischen dem betreffenden Ultraschallsender und dem zugeordneten Ultraschallempfänger zwischen einem und einhundert Zentimetern beträgt.

## Claims

1. Method of measuring in line by the use of at least one ultrasound transmitter (1) and at least one ultrasound receiver (2) acting as an ultrasound sensor the setting characteristics of confectionery/chocolate products placed into moulds in order to determine during the cooling process the optimum time for extracting the finished product from the moulds, wherein no direct contact is made with the confectionery/chocolate product, and wherein the amplitude attenuation (11, 14) of the empty mould (3) is determined from the vibration signal (7) transmitted and received by the ultrasound receiver (2), wherein a mould-specific calibration signal is stored in a process computer or external computer, whereupon the mould (3) is used for the moulding process of the confectionery/chocolate product and the ultrasound (14) sent out from the ultrasound transmitter (1) through the mould material and the moulding material in the form of a confectionery/chocolate product is detected by the ultrasound receiver (2) and whereupon from the dissipative amplitude attenuation and the phase shift (12) of the ultrasound from maximum (7) the relevant time for completely detaching the confectionery/chocolate product from the mould wall is delimited and whereupon on the basis of the subsequently pronounced, progressive decrease in the amplitude signal the time for demoulding the confectionery/chocolate product is determined.

2. Method in accordance with claim 1, **characterised in that** the variations in amplitude and temperature functions over time are transmitted almost instantaneously from the moulding process straight to a process computer or external computer by means of a wireless link.

3. Method in accordance with claim 1, **characterised in that** the variations in amplitude and temperature functions from the moulding process over time are temporarily stored on an electronic chip and after removal of the measuring mould from the moulding process - after demoulding - the data are read out by means of a computer via a cable link.

4. Method in accordance with claim 1 or any one of the following claims, **characterised in that** the data obtained in line on the hardening-setting characteristics of confectionery/chocolate masses, wall-detachment characteristics and the time of complete detachment from the walls are used to control or regulate the process parameters affecting the setting process such as cooling temperature, cooling-air speed and belt speed to achieve a minimum dwell time in the process and/or to optimise the quality of the product properties.

5. Device for applying the method in accordance with any one of claims 1 to 4, having a mould (3) for confectionery/chocolate products as moulding material, at least one ultrasound transmitter and at least one ultrasound receiver (2) for receiving the vibration partly attenuated by the mould material and by the confectionery/chocolate mass in contact with it, where the ultrasound transmitter and the ultrasound receiver are arranged on the mould wall delimiting the hardening moulding material, and having a process computer or external computer set up to apply the aforementioned method.

6. Device in accordance with claim 5, **characterised in that** the excitation vibration frequency of the ultrasound transmitter concerned is tuned to the resonance frequency of the wall delimiting the hardening confectionery/chocolate mass.

7. Device in accordance with claim 5 or 6, **characterised in that** the ultrasound transmitter used and the assigned ultrasound receiver are set between one and one hundred centimetres apart.

## Revendications

1. Procédé destiné à mesurer en ligne, pendant le process de refroidissement, la réaction de solidification de produits chocolatés/de confiserie coulés dans des moules afin de déterminer l'instant optimal où démouler le produit fini, en utilisant au moins un émetteur ultrasonique (1) et au moins un récepteur ultrasonique (2) officiant de capteur ultrasonique, sachant qu'il n'y a pas de contact direct avec le produit chocolaté/de confiserie et sachant qu'à partir du signal oscillant émis et à partir du signal oscillant (7) reçu par le récepteur ultrasonique (2) est déterminée l'atténuation d'amplitude (11, 14) du moule (3) vide, sachant qu'un signal de calibration spécifique au moule est enregistré dans un calculateur de process ou un ordinateur, ce après quoi le moule (3) est mis en place pour le process de moulage du produit chocolaté/de confiserie et ce après quoi l'ultrason (14) se dispersant depuis l'émetteur ultrasonique (1) via le matériau du moule et via la matière moulée en forme de produit chocolaté/de confiserie est détecté par le récepteur ultrasonique (2) et ce après quoi l'instant pertinent de décollement complet du produit chocolaté/de confiserie de la paroi du moule est délimité à partir de l'atténuation d'amplitude par dissipation et à partir du décalage de phase (12) de l'ultrason depuis son maximum (7) et ce après quoi est déterminé, en raison de la diminution subséquente nette en progression constante du signal d'amplitude, l'instant de démoulage du produit chocolaté/de confiserie.

2. Procédé selon la revendication 1, **caractérisé en ce que** les courbes temporelles des fonctions d'amplitude et de température sont transmises sans fil et pratiquement sans retard, depuis le process de moulage, directement à un calculateur de process ou à un ordinateur externe.

3. Procédé selon la revendication 1, **caractérisé en ce qu'**un enregistrement temporaire des courbes temporelles des fonctions d'amplitude et de température provenant du process de moulage a lieu sur un module électronique et qu'après que le moule de mesure a été retiré du process de moulage - après le démoulage -, une liaison par câble permet de lire les données au moyen d'un ordinateur.

4. Procédé selon la revendication 1 ou l'une des revendications suivantes, **caractérisé en ce que** les données déterminées en ligne de la réaction de figeage et solidification des pâtes chocolatées/de confiserie, la réaction de décollement des parois et l'instant de décollement complet des parois sont utilisés pour piloter ou réguler les paramètres processuels influençant le process de solidification tels que la température de refroidissement, la vitesse de l'air de refroidissement et la vitesse du tapis, pour obtenir un temps de séjour minimal dans le process et/ou pour optimiser la qualité des caractéristiques du produit.

5. Dispositif pour réaliser le procédé selon l'une des revendications 1 à 4, comprenant un moule (3) destiné à des produits chocolatés/de confiserie constituant la matière moulée, au minimum un émetteur ultrasonique et au moins un récepteur ultrasonique (2) pour recevoir l'oscillation en partie atténuée par le matériau du moule et par la pâte chocolatée/de confiserie qui le touche, sachant que l'émetteur ultrasonique et le récepteur ultrasonique sont disposés contre la paroi du moule limitant la matière moulée en train de se figer, et comprenant un calculateur de process ou un ordinateur externe configuré pour exécuter le procédé indiqué.

6. Dispositif selon la revendication 5, **caractérisé en ce que** la fréquence d'oscillation excitatrice de l'émetteur ultrasonique concerné est harmonisée avec la fréquence de résonance de la paroi limitant la pâte chocolatée/de confiserie en train de se figer.

7. Dispositif selon la revendication 5 ou 6, **caractérisé en ce que** la distance entre l'émetteur ultrasonique concerné et le récepteur ultrasonique qui lui est affecté est comprise entre un et cent centimètres.
